# EUROPEAN PATENT APPLICATION

(11) **EP 2 098 210 A1**
(43) Date of publication of application: **09.09.2009**
(21) Application number: 09154287.8
(22) Date of filing: 04.03.2009
(51) Int. Cl.: A61H 23/04

(54) **Compression device having an inflatable member with a pocket for receiving a counterforce component**

(30) Priority: 04.03.2008 US 41837
(71) Applicant: Tyco Healthcare Group LP, Mansfield, MA 02048 (US)
(72) Inventor: Nardi, Steven, Taunton, MA 02780 (US); Bock, Malcolm G., Medfield, MA 02052 (US)
(74) Representative: Körber, Martin Hans

(57) **Abstract**

A compression foot cuff includes an inflatable member (14,114). The inflatable member defines a pocket (40) receiving a generally rigid counterforce component.

## Description

### FIELD OF THE INVENTION

The present invention generally relates to a compression device, and more particularly to a foot cuff including an inflatable member with a pocket for receiving a counterforce component.

### BACKGROUND

Compression devices for applying compressive forces to a selected area of a wearer's anatomy are generally employed to improve blood flow in the selected area. Compression devices that provide intermittent pulses of a compressed fluid (i.e. air) to inflate at least one inflatable chamber in a cuff or sleeve are particularly useful. This cyclic application of pressure provides a non-invasive method of prophylaxis to reduce the incidence of deep vein thrombosis (DVT), and the like. These compression devices find particular use during surgery on patients with high-risk conditions such as obesity, advanced age, malignancy, or prior thromboembolism. Patients who develop this condition often have swelling (edema) and tissue breakdown (venous stasis ulcer) in the lower leg. When a DVT occurs, the valves that are located within the veins of the leg can be damaged, which in turn can cause stasis and high pressure in the veins of the lower leg.

Generally, these compression devices are fluidly coupled to a source of pressurized fluid by one or more air tubes. Additionally, each compression device includes a flexible shell having one or more bladders disposed therein. The compression device is placed around the patient's foot or other selected portion whereupon a pressurized fluid is delivered into the bladder creating pressure at the part or parts of the body in contact with the bladder.

Compression cuffs adapted for use with a patient's foot may be used by themselves or combined with one or more additional compression cuffs or sleeves that are disposed on portions of a patient's leg for improving the treatment regimen. In general, each of the additional compression sleeves includes a plurality of separate inflatable chambers that are progressively arranged along a longitudinal axis of the sleeve from a lower portion to an upper portion of the limb. A pressure source, e.g. a controller, is provided for intermittently forming a pressure pulse within these inflatable chambers from a source of pressurized fluid during periodic compression cycles. The compression sleeves provide a pressure gradient along the patient's limbs during these compression cycles which progressively decreases from the lower portion to the upper portion of the limb (e.g. from the ankle to the thigh).

Compression cuffs that are adapted for use with a patient's foot generally include a heel strap with a tab portion that is adapted to fit around a portion of the patient's heel. This arrangement allows the compression cuff to be wrapped around and releasably attached to the patient's foot. The compression cuff may include a generally rigid sole to direct expansion of the inflatable chamber toward the wearer's foot. The rigid sole needs to be located under that portion of the inflatable that is acting on the portion of the foot to produce blood flow out of the foot. Conventionally, the rigid sole is temporarily attached to the bladder by double stick tape. Final location and positioning of the rigid sole may be carried out by stitching. For example, the bladder is typically stitched to an outer wrap of the foot cuff. The stitching can be arranged so that it captures the rigid sole in position relative to the bladder, as well as the outer wrap. This requires care and precision in manufacturing the foot cuff.

Examples of compression cuffs are disclosed in U.S. Pat. Nos. 4,013,069 and 4,030,488 to Hasty, U.S. Pat. Nos. 4,029,087 and 5,795,312 to Dye, U.S. Pat. No. 5,626,556 to Tobler et al., and U.S. Pat. App. Serial No. 11/761,212 to Meyer et al., all of which are currently owned by Tyco Healthcare Group LP and are incorporated by reference herein in their entireties. Other examples of compression cuffs are disclosed in U.S. Patent Nos. 4,696,289 to Gardner et al., 5,989,204 to Lina and 5,345,260 to Cook. An example of compression treatment method is disclosed in U.S. Pat. No. 6,231,532 to Watson et al., which is owned by Tyco Healthcare Group LP, the contents of which are hereby incorporated by reference herein in their entirety.

### SUMMARY

In one aspect, a compression foot cuff for applying compression to a foot of a wearer generally comprises an inflatable member including first and second of fluid impermeable layers secured to one another to define an inflatable chamber. The foot cuff comprises a generally rigid counterforce component. The inflatable member is formed to define a pocket receiving the counterforce component therein.

In another aspect, a method of making a foot cuff device for applying compression to a foot of a wearer generally comprises forming a bladder by joining together generally opposed first and second layers of fluid impermeable material to form a chamber. A generally rigid counterforce component is positioned in a pocket defined by the bladder so that the counterforce component is held in a selected orientation relative to the bladder in the pocket.

Other objects and features will be in part apparent and in part pointed out hereinafter.

### BRIEF DESCRIPTION OF THE DRAWINGS

FIG. 1 is an exploded perspective of a first embodiment of a compression foot cuff in accordance with the present disclosure;

FIG. 2 is a perspective of a bladder of the foot cuff with a sole attached thereto;

FIG. 3 is an exploded view of FIG. 2;

FIG. 4 is a section of the bladder with the attached sole taken along the line 4--4 in FIG. 2;

FIG. 4A is an exploded view of FIG. 4;

FIG. 5 is a perspective of a second embodiment of a bladder of a foot cuff with a sole attached thereto, an outer layer of the bladder being broken away to show the underlying sole; and

FIG. 6 is an exploded view of the bladder in FIG. 5

Corresponding reference characters indicate corresponding parts throughout the drawings.

### DETAILED DESCRIPTION OF THE DRAWINGS

Referring now to the drawings, a compression foot cuff for applying compressive pressure to a wearer's foot is generally indicated at 10. The foot cuff is adapted for use in a compression therapy system, which further includes a supply of pressurized air (not shown) and tubing connecting the supply of pressurized air to the foot cuff.

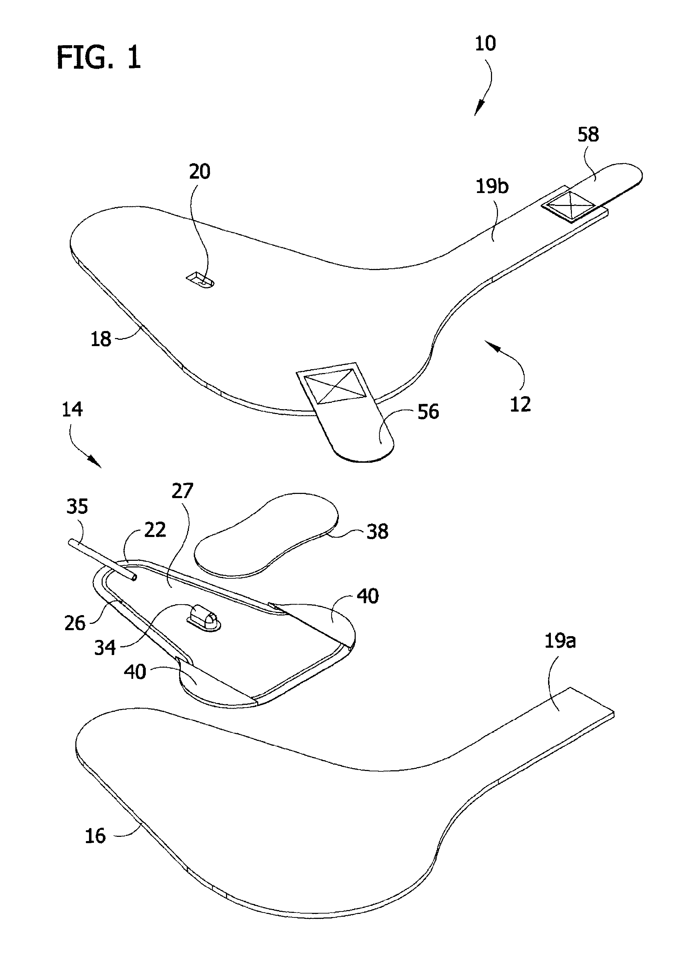

As shown best in FIG. 1, the foot cuff 10 includes an envelope, generally indicated at 12, substantially enveloping or enclosing a bladder, generally indicated at 14. The envelope 12 includes an inner contact layer 16 and an outer layer 18 secured to one another generally adjacent to corresponding perimeters of the layers to define an interior space for receiving and substantially enclosing the bladder 14 (broadly, "an inflatable member") therein. The contact layer 16 and the outer layer 18 may be fixedly secured to one another at their peripheries, such as by heat welding, adhesives, sewing or other suitable ways. Alternatively, the contact layer 16 and the outer layer 18 may be releasably secured to one another. In use the contact layer 16 is adjacent to the wearer's foot and the outer layer 18 is located farthest from the foot. As used herein, the terms "inner" and "outer" indicate relative positions of respective components and surfaces with respect to the skin of the wearer's body part when the compression device is secured to the body part, and as such, an "inner" component or surface is more adjacent to the skin of the body part than an "outer" component or surface.

Contact layer 16 and outer layer 18 of the envelope 12 include ankle strap portions 19a and 19b respectively. Ankle strap portions 19a, 19b have a longitudinally projecting configuration for wrapping about a portion of the foot adjacent to the ankle. The ankle strap portions 19a, 19b can be sewn, RF welded, or sonic welded. However, in the illustrated embodiments, the ankle strap portions 19a, 19b are formed as one piece with the contact layer 16 and outer layer 18, respectively.

Contact layer 16 of the envelope 12 is adapted for contacting the foot. Contact layer 16 is in one embodiment fabricated from a chemically treated material, with wicking ability, for wicking away moisture from the skin. In one embodiment, contact layer 16 includes a mesh-like fabric capable of wicking moisture away from the patient's skin. Furthermore, the contact layer 16 can be faced with a soft material toward the treatment surface of the patient. For example, the material can be a thin layer of open celled porous foam, napped cloth, or a layer of vapor permeable cloth permeable. It is understood that the cuff 12 may not include a contact layer within the scope of the present invention.

Outer layer 18 of the envelope 12 includes an opening 20 for permitting a pressurized fluid inlet passage therethrough. Outer layer 18 is configured for providing the attachment surface for a hook and loop feature of cuff 12, as will be described in more detail herein below. Moreover, the outer layer 18 provides a soft material for cushioning effect against the top portion of the feet and may be fabricated from similar materials as contact layer 16 and in similar dimensions therewith for corresponding geometry. Alternatively, outer layer 18 may be fabricated from a laminated material, such as, for example, sontara fabric, open cell urethane foam, or loop fabric. It is understood that the cuff 12 may not include an outer layer within the scope of the present invention.

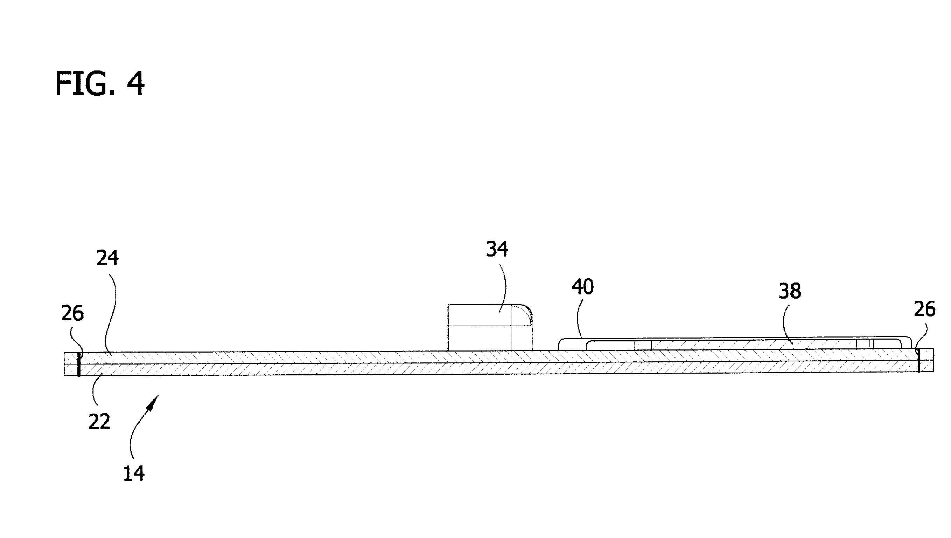

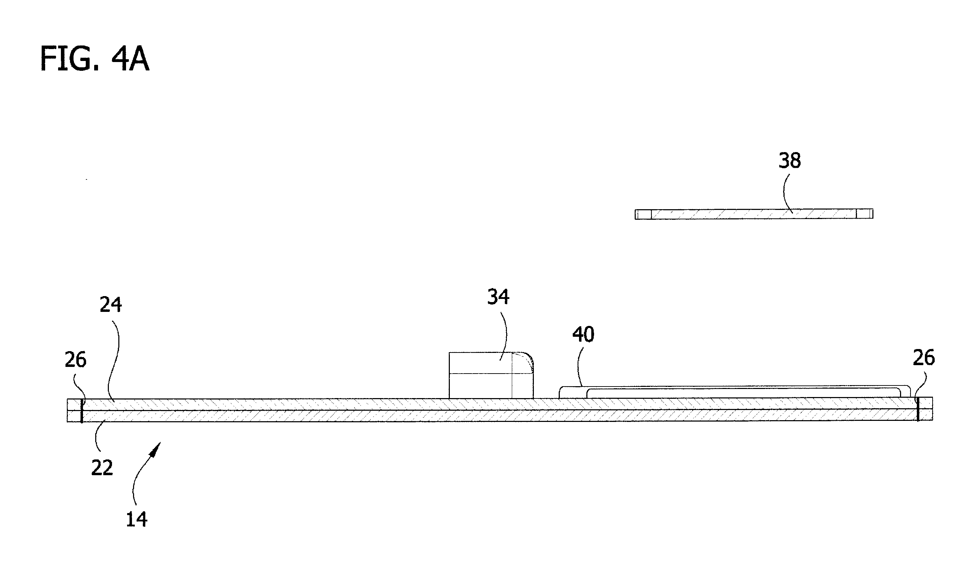

The bladder 14 is configured for positioning against the bottom portion of the foot. Referring to FIG. 4, bladder 14 includes outer and inner layers 22, 24 of air impermeable material (e.g., PVC) joined together in a suitable manner along a line 26 adjacent to their peripheries to define a single inflatable chamber 27. The layers 22, 24 may be joined to one another such as by radio frequency (RF) welding. Other ways of joining the layers 22, 24 include sewing, adhesive, heat sealing, etc. It is understood that the bladder 14 can include more than one inflatable chamber 27 within the scope of the present invention. The inflatable chamber 27 of the bladder 14 is adapted for receiving and retaining a pressurized fluid (e.g. air) for exerting compressive pressure to the foot during successive pressure application cycles. The inflatable chamber 27 has an inlet member 34 (broadly, a port) and a tube 35 connected to the inlet member for air or fluid to be introduced into the chamber during the start of a compression cycle and to be exhausted to end the compression cycle. The inlet member 34 of the illustrated embodiment is a plastic component that is secured such as by heat welding or other means to the bladder 14. It is understood that other ways of introducing air or fluid into the chamber 27 are within the scope of the invention.

A rigid sole (broadly, a counterforce component), generally indicated at 36, is disposed between the outer layer 18 of the envelope 12 and the outer layer 22 of the bladder 14. It is believed the sole 38 provides a counterforce to the outer layer 22 of the bladder 14 as the bladder is expanding to direct expansion toward the contact layer 16 and the user's foot. In this way, the inner layer 24 expands outward more than the outer layer 22 to direct compressive force toward the user's foot. The sole 38 may be constructed from a polypropylene material or other material within the scope of the invention.

The generally rigid sole 38 is received in a pair of spaced apart pockets 40 formed on the outer layer 22 of the bladder 14 to fix the position of the sole relative to the bladder. The pockets 40 are formed by attaching respective pieces of material to the outer layer 22 of the bladder 14. For example, the pieces of material may be of the same material as the outer layer 22 of the bladder 14 (e.g., PVC) or may be of different material than the outer layer. The pieces of material forming the pockets 40 may be heat-welded or attached to the bladder 14 in other ways, such as by adhesives and sewing. In one example given without limitation, the sole 38 is inserted into the pockets 40 after the pieces of material are attached to the bladder 14. In another example given without limitation, the sole 38 is properly positioned relative to the bladder 14 and the pieces of material forming the pockets 40 are attached to the bladder with sole in position. Although the illustrated embodiment includes two pockets 40 receiving the sole 38, it is understood that the foot cuff 10 may have one or more than two pockets within the scope of the invention. For example and without being limiting, a single pocket may be formed by placing a single piece of material over the sole and attaching the single piece of material to the outer layer of the bladder to trap the sole between the single piece of material and the outer layer.

Hook fasteners 56, 58 are provided for securing the wrapped cuff 12 around a foot, and are positioned on the outer layer 18 of the cuff. Hook fastener 56 is mounted to strap portion 19b of outer layer 18 of foot cuff 12 while hook fastener 58 is mounted on a surface of outer layer 18. In use, when ankle strap portions 19a, 19b are wrapped about the back of the foot, hook element 56 engages outer layer 18 to facilitate mounting of foot cuff 12 on the foot. An identification tab (not shown) may also be included for providing information such as the model number and manufacturer name. Hook fasteners 56, 58 may have tabs (not shown) without fastening material thereon to provide convenient gripping locations on the hook fasteners to thereby allow the practitioner to easily remove the hooks from the outer face 18b of outer layer 18. The use and operation of the foot cuff 12 for applying compression therapy to the wearer's foot is generally known in the art and will not be described herein.

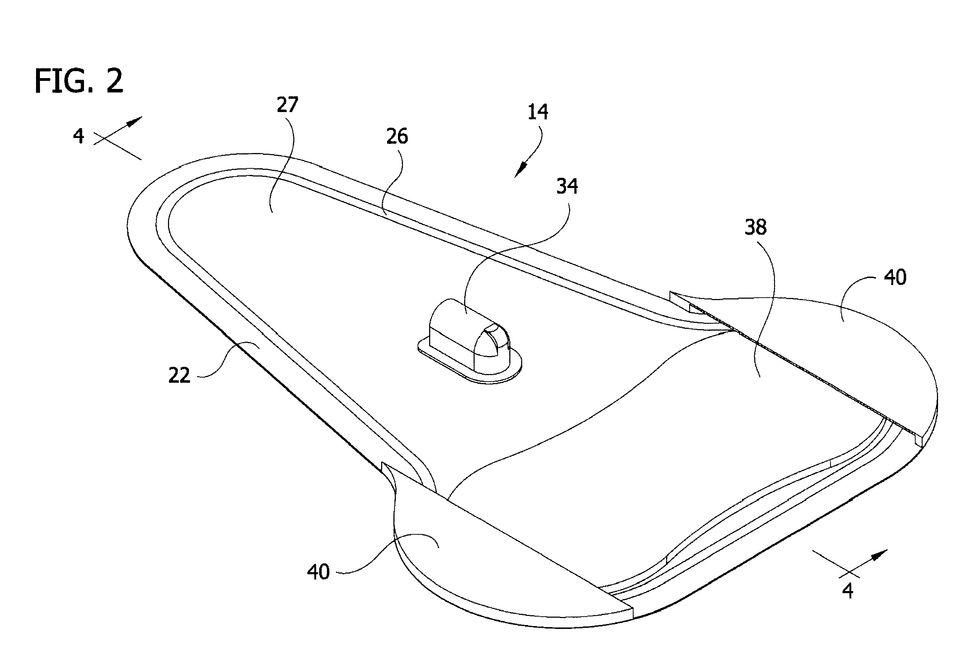

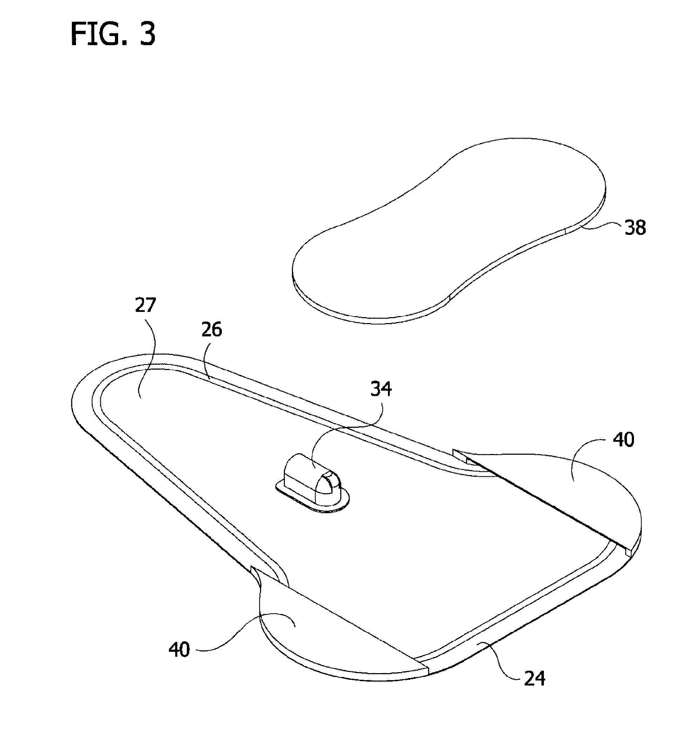

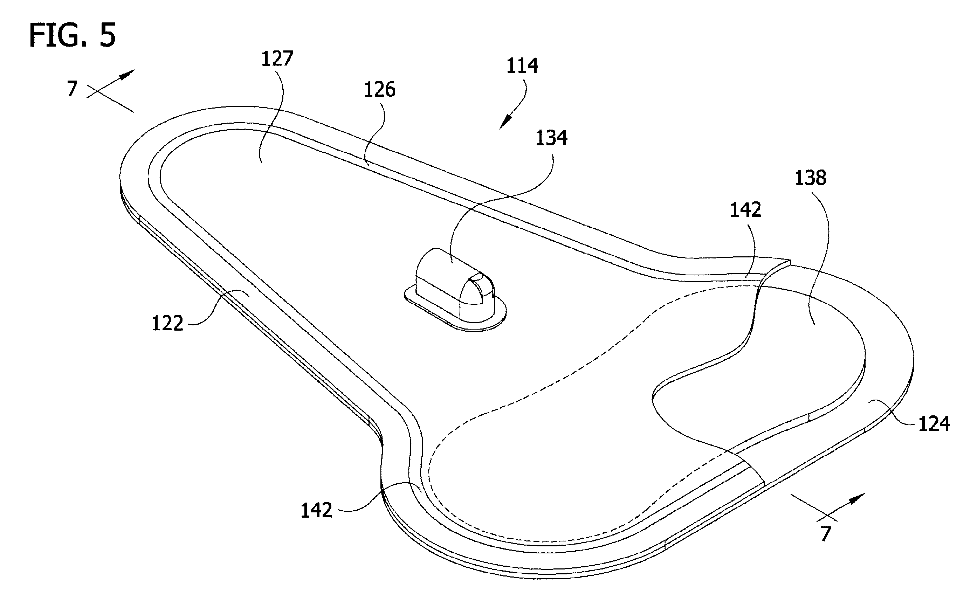

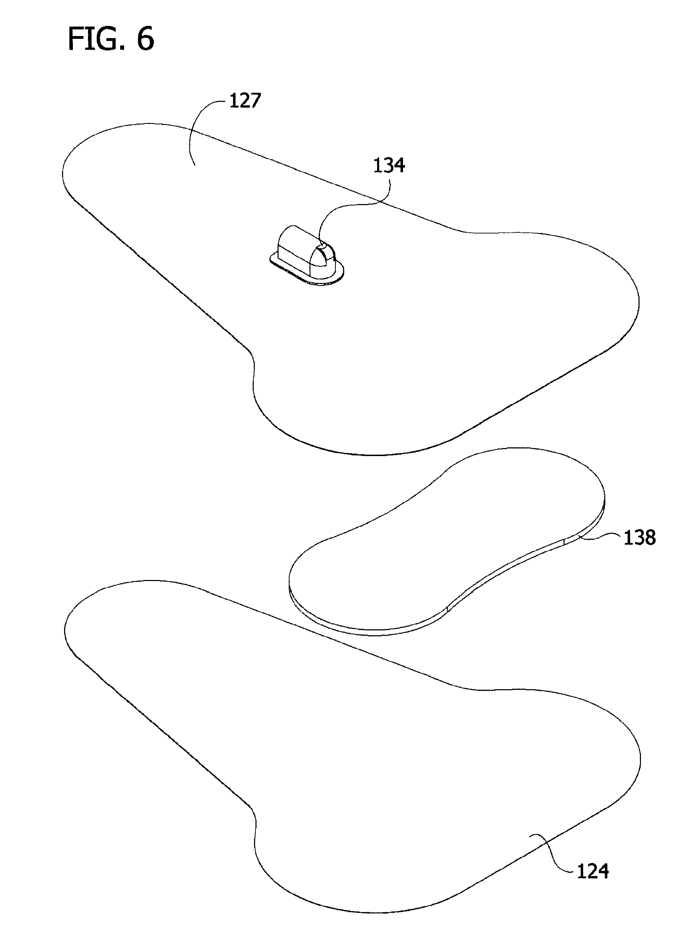

Referring to FIGS. 5-6, another embodiment of a foot cuff 110 is similar to the foot cuff of FIGS. 1-4, and therefore, like components are indicated by corresponding reference numerals, plus 100. In the present foot cuff 110, opposing portions 142 of the line 126 defining the inflatable chamber 127 are sized and shaped to capture the sole 138 within the chamber. The portions 142 of the line 126 extend partially around the perimeter of the sole 138 in conformance with the shape of the adjacent part of the sole to fix the position of the sole relative to the bladder. For purposes of this embodiment, the portions 142 define pockets receiving the sole 138. In one example without being limiting, the sole 138 is attached to the outer layer 122 within the inflatable chamber 127 to further fix the position of the sole relative to the bladder 114 and to further inhibit expansion of the bladder toward the outer layer.

Having described the invention in detail, it will be apparent that modifications and variations are possible without departing from the scope of the invention defined in the appended claims.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above constructions, products, and methods without departing from the scope of the invention, it is intended that all matter contained in the above description and shown in the accompanying drawings shall be interpreted as illustrative and not in a limiting sense.

## Claims

1. A compression foot cuff for applying compression to a foot of a wearer, the foot cuff comprising:
an inflatable member including first and second of fluid impermeable layers secured to one another to define an inflatable chamber;
a generally rigid counterforce component;
the inflatable member being formed to define a pocket receiving the counterforce component therein.

2. A compression foot cuff as set forth in claim 1 wherein the counterforce component is received in the chamber and the pocket comprises a portion of the chamber shaped to capture the counterforce component and hold it in place with respect to the inflatable member.

3. A compression foot cuff as set forth in claim 2 wherein the counterforce component is attached to one of the first and second layers of the inflatable member.

4. A compression foot cuff as set forth in claim 1, 2 or 3 further comprising a piece of material attached to an outer surface of one of the first and second layers to form the pocket.

5. A compression foot cuff as set forth in claim 4 further comprising another piece of material attached to the outer surface of said one of the first and second layers to define another pocket, generally opposite ends of the counterforce component being received in respective pockets.

6. A compression foot cuff as set forth in claim 4 or 5 wherein the pieces of material are heat-welded to the first fluid impermeable layer of the inflatable member.

7. A compression foot cuff as set forth in claim 4, 5 or 6 wherein the pieces of material are the same material as the first and second layers.

8. A method of making a foot cuff device for applying compression to a foot of a wearer, the method comprising:
forming a bladder by joining together generally opposed first and second layers of fluid impermeable material to form a chamber;
positioning a generally rigid counterforce component in a pocket defined by the bladder so that the counterforce component is held in a selected orientation relative to the bladder in the pocket.

9. A method as set forth in claim 8 wherein positioning the counterforce component comprises inserting the counterforce component into the chamber formed by joining the first and second layers.

10. A method as set forth in claim 9 further comprising securing the counterforce component to one of the first and second layers in the chamber.

11. A method as set forth in claim 8, 9 or 10 further comprising securing a piece of material to the first layer of fluid impermeable material to form the pocket on the first layer of fluid impermeable material.

12. A method as set forth in one of the claims 8 to 11 wherein positioning the counterforce component comprises inserting the counterforce component in the pocket after the pocket is formed on the first layer of impermeable material.

13. A method as set forth in one of the claims 8 to 12 wherein the counterforce component is received in the pocket simultaneously as the pocket is being formed.

14. A method as set forth in one of the claims 8 to 13 further comprising securing another piece of material to the first layer to form another pocket, opposite ends of the counterforce component being received in respective pockets.

## Amended claims

### Amended claims in accordance with Rule 137(2) EPC.

**1.** A compression foot cuff (10) for applying compression to a foot of a wearer, the foot cuff (10) comprising:
an inflatable member (14) including first and second of fluid impermeable layers (22, 24) secured to one another to define an inflatable chamber (27); and
a generally rigid counterforce component (38) for providing a counterforce to the expanding inflatable member (14) and for direction expansion towards the foot of the wearer;
**characterized in**
**that** the inflatable member (14) comprises a pocket (40) having the counterforce component (38) positioned therein and
**that** a piece of material is attached to an outer surface of one of the first and second layers (22, 24) to form the pocket (40).

**2.** A compression foot cuff (10) as set forth in claim 1 further comprising another piece of material attached to the outer surface of said one of the first and second layers (22, 24) to define another pocket (40) , generally opposite ends of the counterforce component (38) being received in respective pockets (40).

**3.** A compression foot cuff (10) as set forth in claim 1 or 2 wherein the pieces of material are heat-welded to the first fluid impermeable layer (22) of the inflatable member (14).

**4.** A compression foot cuff (10) as set forth in claim 1, 2 or 3 wherein the pieces of material are the same material as the first and second layers (22, 24).

**5.** A method of making a foot cuff device (10) for applying compression to a foot of a wearer, the method comprising:
forming a bladder (14) by joining together generally opposed first and second layers (22, 24) of fluid impermeable material to form a chamber (27);
securing a piece of material to an outer surface of one of the first and second layers (22, 24) to form a pocket (40); and
positioning a generally rigid counterforce component (38) for providing a counterforce to the expanding bladder (14) and for direction expansion towards the foot of the wearer in the pocket (40) so that the counterforce component (38) is held in a selected orientation relative to the bladder (14) in the pocket (40).

**6.** A method as set forth in claim 5 comprising securing a piece of material to the first layer (22) of fluid impermeable material to form the pocket (40) on the first layer (22) of fluid impermeable material.

**7.** A method as set forth in one of the claims 5 or 6, wherein positioning the counterforce component (38) comprises inserting the counterforce component (38) in the pocket (40) after the pocket (40) is formed on the first layer (22) of impermeable material.

**8.** A method as set forth in one of the claims 5 to 7 wherein the counterforce component (38) is received in the pocket (40) simultaneously as the pocket (40) is being formed.

**9.** A method as set forth in one of the claims 5 to 8 further comprising securing another piece of material to the first layer (22) to form another pocket (40), opposite ends of the counterforce component (38) being received in respective pockets (40).
